# EUROPEAN PATENT APPLICATION

(11) **EP 1 295 615 A1**
(43) Date of publication of application: **26.03.2003**
(21) Application number: 02256400.9
(22) Date of filing: 16.09.2002
(51) Int. Cl.: A61L 31/18, A61L 31/04

(54) **Radiopaque stent**

(30) Priority: 19.09.2001 US 955636
(71) Applicant: Cordis Corporation, Miami Lakes Florida 33014 (US)
(72) Inventor: Carpenter, Erin, Harrisville, Rhode Island 02830 (US)
(74) Representative: Belcher, Simon James

(57) **Abstract**

A stent system comprises a stent made of a conventional metal alloy, such as stainless steel, treated with chains of peptides ("anchors") which comprise functionality to bind to the metal surface and to bind to other peptides (which other peptides are termed "recognition" peptides) and recognition peptides, which comprise one end which binds to ("recognizes") the anchor peptide and one end which is bound to a radiodense dye, which system enhances radiopacity relative to the stent alone.

## Description

The present invention generally relates to a stent system, especially stents and covered stents. More particularly, the present invention is directed to stents with enhanced radiopacity generated by the use of anchors and tags.

Blood vessels in the body may become narrowed, and the flow of blood impeded. Such a condition is referred to as a stenosis or a stenotic condition. One way of alleviating problems associated with the stenosis is to insert a stent to help widen the narrowed passage by supporting the walls of the vessel to keep it open.

In placing and monitoring of the stent, it is desirable to be able to visualize the position of the stent within the body. A typical method of visualization is fluoroscopy, which involves the use of a continuous x-ray beam in real time with live images being displayed on a monitor. Although representative stents are made of stainless steel, which generally can be visualized by x-rays, the small dimensions of stents, particularly coronary stents, are such that there is difficulty in visualizing the stents via fluoroscopy. The present invention is directed to a stent system wherein the ability to visualize the stent in the body by a technique such as fluoroscopy is enhanced. To fully appreciate the scope of the invention, background on stents is provided.

Generally, stents, grafts, and graft stents are implantable medical devices (sometimes termed implantable tubular prostheses) which are placed within blood vessels and other body passageways to treat disease conditions such as stenoses, occlusions, and aneurysms. Transluminal implantation of such devices requires that they be introduced to the site collapsed about or within an introduction device and released to self expand or are expanded by other mechanisms to an expanded tubular state providing a lumen of approximately the same size as the patent vessel or duct lumen.

Typically, stents are made from a metal alloy, such as, but not limited to, stainless steel, and have a hollow tubular shape. To meet requirements for medical use, more fully discussed below, the stents have an open lattice-like structure, in which the individual metal components, such as struts, have a diameter or thickness of 0.076 mm (0.003 inch) or less. This small dimension renders the strut difficult to detect in techniques employing x-radiation ("x-rays"), such as fluoroscopy.

Scattering of x-rays is approximately proportional to the square of atomic number, so that materials of atomic number higher than the components of the metal alloy of the stent would enhance scattering, and detectability. However, higher atomic number materials tend to be more expensive and more difficult to fabricate than stainless steel. One approach is to coat the stent, comprising a typical metal alloy such as steel, with a metal of higher atomic number. However, there are concerns associated with corrosion through galvanic effects. The present invention is directed to a stent system which has enhanced radiopacity by the use of "anchors" to the metal surface (which anchors are chains of peptides that adhere to the metal implant surface) and tags (which is connected to the anchor and which achieve high contrast in the visualization medium employed). Such a system employing anchors and tags does not manifest corrosion through galvanic effects. Before discussing this further, a review of stent use and construction is provided.

When the body lumen is weakened, for example, dissectional artery lining occurs in a body lumen such as a blood vessel, the weak part of the body lumen might collapse to occlude a fluid passageway. To prevent such an occlusion, a stent is implanted within the blood vessel to support the blood vessel from the inside thereof. Namely, a stent is delivered to a desired location in the blood vessel, and expanded in a circumferential direction in the blood vessel to support and maintain the patency of the blood vessel. Using the stent to support the blood vessel can avoid surgical exposing, incising, removing, replacing or bypassing a defective blood vessel required in the conventional vascular surgery.

Stents can be viewed as scaffolding, of generally cylindrical symmetry, that function to physically support, and, if desired, expand the wall of the passageway. Typically, a stent consists of two or more struts or wire support members connected together into a lattice-like or open weave frame. Most stents are compressible for insertion through small cavities, and are delivered to the desired implantation site percutaneously via a catheter or similar transluminal device. Once at the treatment site, the compressed stent is expanded to fit within or expand the lumen of the passageway. Stents are typically either self-expanding or are expanded by inflating a balloon that is positioned inside the compressed stent at the end of the catheter. Intravascular stents are often deployed after coronary angioplasty procedures to reduce complications, such as the collapse of arterial lining, associated with the procedure.

There have been introduced various types of stents, and they can be typically categorized from viewpoints of methods for expanding the stent, shapes, methods for manufacturing the stent, designs and so on. From a viewpoint of methods for expanding the stent, stents can be categorized as a self-expandable stent which can be expanded by itself, and a balloon expandable stent. In the balloon expandable stent, the stent is mounted on an expandable member, such as a balloon, provided on a distal end of an intravascular catheter, and the catheter is advanced to the desired location in the body lumen to deliver the stent. Then, the balloon on the catheter is inflated to expand the stent into a permanent expanded condition, and the balloon is deflated for removing the catheter from the stent.

From a viewpoint of materials, stents can be categorized into a tubular stent and a wire stent, and from a viewpoint of methods for manufacturing the stent, stents can be categorized as an etched stent and a laser cut stent. Then, from a viewpoint of designs, stents can be categorized into numerous types, but roughly, stents can be categorized into a stent having a "coil" pattern on the surface thereof, and a stent having a mesh or "ring" pattern on the surface thereof.

Expandable intraluminal vascular grafts are disclosed in US-4733665, US-4739762, US-4776337 and US- 5102417. The '665 document discloses stents which are expanded using angioplasty balloons. The stents are variously a wire mesh tube or of a plurality of thin bars fixedly secured to each other. The devices are installed, for example using an angioplasty balloon and consequently are not seen to be self-expanding. The '762 and '337 documents disclose the use of thin-walled, biologically inert materials on the outer periphery of the earlier-described stents. The '417 document discloses the use of multiple stent sections each flexibly connected to its neighbour.

In all types of stents, the stent expands from an initial diameter to a larger diameter so as to be suitable for a particular size of the targeted body cavity. Therefore, the stent must be expandable in the circumferential direction. Also, since the reason the stent is placed in the body lumen is to support a cavity wall therein to maintain the patency thereof, it is very important that the stent has radial strength as well as support capability.

At the same time, since the stent is generally delivered through tortuous path to the desired location in the body lumen, the stent must have flexibility in the axial direction. Namely, the stent must be flexible and is bent easily to thereby facilitate the delivery of the stent in the narrow and meandering body lumen.

In the aforementioned various types, since simply bending a wire forms a wire stent, this makes the wire stent not only expanded easily, but also shrunk easily. Namely, the wire stent does not have support capability for maintaining the expanded condition in order to keep the body lumen open. On the other hand, a tube type stent has enough support capability to maintain its expanded condition for holding the body lumen open.

The present invention is directed to a stent system which allows the use of lower cost, more easily fabricated, stents, which are less radiodense, in conjunction with materials which are more radiodense, thereby allowing greater visualization in vivo during catheter introduction into the vessel, stent deployment, and post-operative diagnosis. Accordingly, an object of the invention is to provide a stent system which is sufficiently radiopaque, flexible, has a low profile, is substantially non-thrombogenic, and which will eliminate corrosion.

Another object of the invention is to provide an external surface in the stent system that is both biocompatible and sufficiently scattering to x-rays that the stent system is easily visualized using techniques such as fluoroscopy.

Another object of the present invention is to minimize galvanic corrosion between dissimilar metals.

Basic background material may be found in H Lodish et al, Molecular Cell Biology, 3^{rd} edition, Scientific American Books 1995 and A Guyton and J Hall, Textbook of Medical Physiology, 9^{th} edition, W B Saunders.

The present invention provides a stent system in which the stent is tagged with a probe showing enhanced sensitivity to a given visualization device.

The present invention is generally directed to a stent system which comprises a material more radiopaque than the metals typically used to manufacture stents ("the radiodense material"), which radiodense material is placed within the stent system in such a way as to minimize or reduce corrosion problems associated with galvanic effects. Galvanic effects in the stent system can be minimized by coating the metal of the stent with a material which is not electronically conducting. The radiodense material can be coated onto the non-electronically conducting material.

One embodiment of the present invention is a stent system comprising a stent manufactured of a metal alloy, such as stainless steel, treated with chains of peptides that adhere to the metal alloy surface (denoted "anchors, wherein each anchor peptide has a free end which "recognizes" (or binds) a "recognition" peptide), with the chains of peptides treated with recognition peptides, wherein one end of the recognition peptide entity is attached to a dye molecule ("tag") and the other end is bound by the free end of the anchor peptide.

Embodiments of the present invention will now be described by way of example with reference to the accompanying drawings, in which:

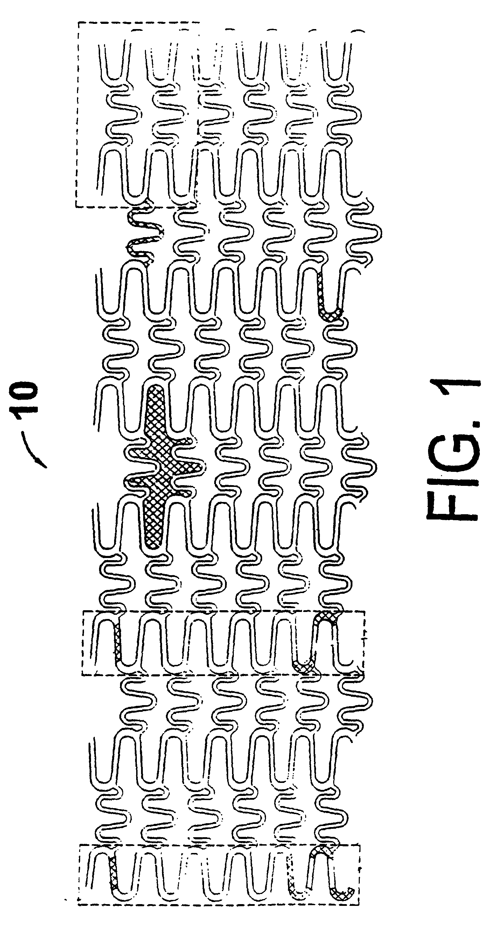

Fig. 1 is a perspective view of a stent; and

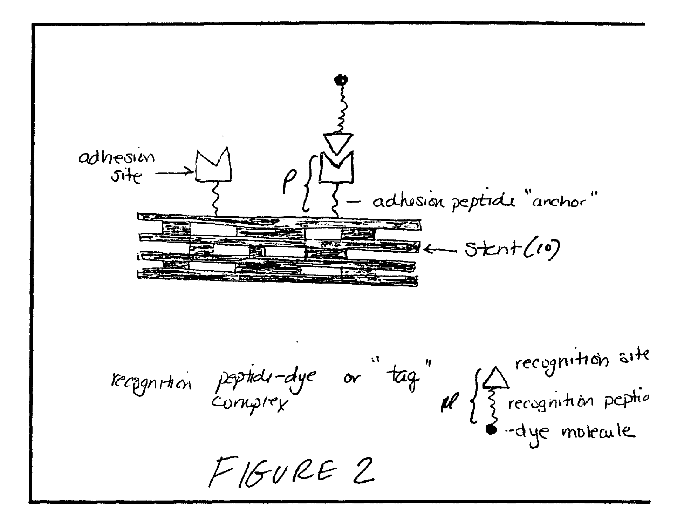

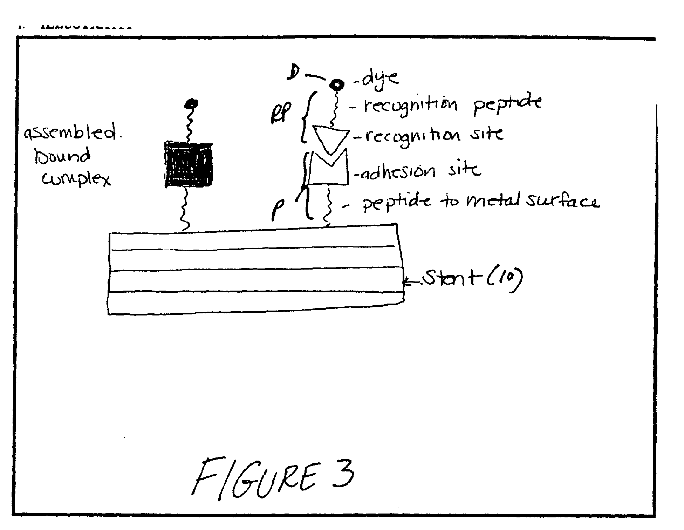

Figs. 2 and 3 are schematic diagrams of the anchor and tab system of the present invention.

When designing endovascular medical devices (especially stents) there is often a need for adequate visualization (for example radiopacity). In the area of stents, it is often necessary to be able to visualize implantable medical devices using a fluoroscope, which employs x-rays. The ability to visualize via x-rays depends both on the scattering power of the material in the stent (scattering power going roughly as the square of the atomic number of the element; x-ray absorption also increasing with atomic number) and the amount of the material (the thicker the material, the more easily visualized). As the density and thickness of a material increase, so does the radiopacity. However, in a move toward less invasive techniques, the thickness of a given component of a stent is limited. Further, with conventional metal alloys (for example stainless steel) at the dimensions used for stents (for example less than 0.076 mm (0.003 inches)), there is difficulty in visualizing stents in fluoroscopy.

The present invention discloses a method for enhancing the radiopacity of stents and a stent system formed from the method. The method for forming the stent system involves first treating a conventional stent comprising metal with an anchoring entity, which anchoring entity may comprise chains of peptides that adhere to the metal of the stent and second treating the stent, comprising the anchoring entity, with a recognition peptide, which recognition peptide is capable of binding to the anchoring entity and which recognition peptide comprises a tag which comprises a dye or contrast medium, which tag enhances the radiopacity of the stent system relative to the stent alone. The stent system comprises a stent 10, peptides P adhering to the surface of the stent ("anchors") which peptides comprise a recognition site R capable of binding to recognition peptides RP, and recognition peptides which are both bound to the anchors and which comprise a dye which enhances the radiopacity of the stent system relative to the stent.

In more detail, in anchor/tag visualization, a metal stent is treated with "anchors" (chains of peptides that adhere to the metal implant surface). The "tag" is a dye/contrast medium which is treated with recognition peptides (one end of a given recognition peptide is attached to the dye molecule and the other end is "recognized" (or bound) by the free end of the anchor peptide. Essentially, the open binding sites on the anchor and tag are stereogenic, exactly complementary by geometry, and bind with high specificity and affinity (in a similar way to an antibody/antigen mechanism). The adhesion of the anchor to the stent ("implant") is important to the visualization of the stent under fluoroscopy because it allows for a place for the dye recognition peptide complex to bind. Once access to the vasculature is gained, the stent is highly radiopaque as the physician checks the progress of the stent to the site of the lesion.

The present invention allows for more exact stent placement through better radiopacity introduced through the tag. The anchor peptides and recognition peptides are biocompatible and degradable. Further, as the peptide-dye complex degrades, it is readily absorbable and would not cause micro-emboli or a foreign body encapsulation response.

In alternate embodiments, the anchor peptides and recognition peptides could be introduced at the same time or could be introduced at different times.

In an alternate embodiment, the number of tags delivered does not correspond exactly to the number of anchors available.

In an alternate embodiment, the anchor peptides may release the tags without themselves becoming dislodged from the device or losing functionality.

The present anchor/tag invention presents advantages over the prior art. The method of the present invention gives physicians an alternative when viewing stents under fluoroscopy. The anchor/tag method of intermittent stent visualization overcomes the traditional barriers of radiopacity, density, and mass of stent material. Past alternative methods of radiopacity have included metallic coatings on stents which both increase the mass and which present corrosion problems. The present method is unique in that impart radiopacity to the device, potentially temporarily and potentially with the ability to degrade over time. In those situations wherein degradation has occurred, the stent can be made radiopaque at a later time. Further, the device can be manufactured with anchor peptides only and then dipped in a solution with the recognition peptide/tag complex at a later time and prior to the medical procedure.

In an alternate embodiment, the recognition peptide/dye complex is degradable over time and/or has the ability to release from the anchor, with the anchor retaining the ability to reattach to another tag complex, if the other tag complex were introduced.

In an alternate embodiment, proteins could release the tags in a few days (for example, two days), correlating to when the patient is released from the hospital. At the time of followup (for example, six months later), the physician could deliver a local bolus of radiopaque tag solution endovascularly, which would bind to anchor peptides. This process can be repeated multiple times until device becomes completely encapsulated by the body.

Relevant to prior art stent coatings, the anchor/tag method of stent visualization is not constrained by the density and mass of stent material as in traditional approaches to stent visualization. Current alternate methods include the use of metallic coatings on stents to enhance radiopacity. However, metallic coatings often rely on the use of dissimilar metals, which may result in a galvanic effect releasing metallic ions into the blood stream and surrounding tissues.

This proposed anchor/tag method does not rely on dissimilar metals and uses naturally occurring amino acids. Proteins have enhanced biocompatibility. Proteins also lend themselves to endothelialization and promote healing at the site of implantation. The use of this invention to enhance radiopacity results in a lower profile (because of reduced wall thickness) and more flexible stents. Thus, the amount of material needed to make a stent adequately visible under fluoroscopy can be reduced.

The present invention has further embodiments. The stent could be alternatively treated on inside diameter, outside diameter, or both diameters, depending on the level of radiopacity desired. Different dyes and anchors could be used for different applications (for example, biliary, arterial, venus, etc.) depending upon tissue morphology. The treatment or coating can be placed on the device prior to sterilization and packaging or as a part of the procedure. For example, the clinician could submerge the device in one solution to attach anchor peptides and then a second solution for attachment of the recognition peptide-dye complex. Alternatively, the stent could be provided pre-coated with anchor peptides and the clinician could submerge in solution containing tags. The intermittent radiopacity can be classified into three, roughly defined, categories: short term (for example about two days), mid-term (for example about one month) and long term (for example about six months).

The anchor/tag method of stent visualization of the present invention is related to work in immuno-fluorescence, although the immuno-fluorescence work involves different wavelengths of observation and different means of visualization.

## Claims

1. A stent system which comprises a metallic stent treated with anchor chains of peptides which bind to the metal surface and recognition peptides, which comprise one end which binds to the anchor peptide and one end which is bound to a radiodense dye.

2. The stent system of claim 1 wherein the metal is stainless steel.

3. A stent system which comprises a metallic stent treated with chains of peptides which bind to the metal surface and to other peptides.

4. A method of forming a stent system with enhanced visualization in fluoroscopy comprising the steps of:
treating a metallic stent with anchoring peptides which are capable of adhering to the metal surface and are functionality capable of binding to other peptides;
treating a metallic stent treated with the anchoring peptides with recognition peptides, which recognition peptides are functionality capable of binding to the anchoring peptides and capable of binding to radiodense dyes.

5. A method of forming a stent system with enhanced visualization comprising the steps of:
treating a stent with anchoring peptides which are functionality capable of adhering to the stent surface and to other peptides, termed recognition peptides.

6. The method of claim 5 further comprising treating the stent comprising anchoring peptides with recognition peptides which both bind to the anchoring peptide and which comprise an element which causes enhanced visualization relative to the stent alone.
